# EUROPÄISCHE PATENTANMELDUNG

(11) **EP 0 778 033 A2**
(43) Veröffentlichungstag der Anmeldung: **11.06.1997**
(21) Anmeldenummer: 96119251.5
(22) Anmeldetag: 30.11.1996
(51) Int. Cl.: A61M 1/34

(54) **Vorrichtung zur Behandlung von Blut mit Flüssigkeitsbilanzierung**

(30) Priorität: 09.12.1995 DE 19546028
(71) Anmelder: Fresenius AG, D-61350 Bad Homburg (DE)
(72) Erfinder: Beden, Josef, 55252 Mainz-Kastel (DE); Flaig, Hans-Jürgen, Dr., 36341 Lauterbach (DE); Steinbach, Bernd, Dr., 61169 Friedberg (DE)
(74) Vertreter: Fuchs, Luderschmidt & Partner

(57) **Zusammenfassung**

Ein als Folienbeutel ausgebildetes Bilanzierdisposable besteht im wesentlichen aus zwei Folien (1,2), die unter Ausbildung einer durch eine flexible Wand (8) in die beiden Kammerhälften (9, 10) unterteilten Bilanzierkammer (4) und unter Ausbildung von zwei Ausgleichskammern (5, 6) sowie der die einzelnen Kammern verbindenden Kanäle (15 bis 18) miteinander verschweißt sind. Die flexible Wand (8), die die erste Kammerhälfte (9) der Bilanzierkammer (4) von der zweiten Kammerhälfte (10) trennt, wird durch eine Zwischenfolie gebildet, die zwischen die erste und zweite Folie (1, 2) im Bereich des Bilanziervolumens eingelegt und mit den äußeren Folien unter Ausbildung der ersten und zweiten Kammerhälfte (9, 10) druckdicht verschweißt ist. Die feste Volumenvorgabe der Bilanzierkammerhälften (9, 10) wird durch einen entsprechenden Systemeinschub erreicht, in den das Disposable passend eingelegt wird. Der Systemeinschub weist zwei starre Aufnahmekörper (25, 26) mit muldenförmigen Vertiefungen (27) zum Über- bzw. Untergreifen der Bilanzierkammer (4) des Disposables auf. Das Bilanzierdisposable läßt sich in einem einzigen Schweißvorgang kostengünstig herstellen und ist einfach zu handhaben.

## Beschreibung

Die Erfindung betrifft ein Bilanzierdisposable zum Bilanzieren von Flüssigkeiten für eine medizinische Behandlungsvorrichtung sowie eine medizinische Behandlungsvorrichtung mit einem Systemeinschub zur Aufnahme eines derartigen Bilanzierdisposables.

Zur Entfernung von harnpflichtigen Substanzen und zum Flüssigkeitsentzug werden beim chronischen Nierenversagen verschiedene Verfahren zur apparativen Blutreinigung bzw. Blutbehandlung eingesetzt. Bei der Hämodialyse (HD) überwiegt der diffusive Stofftransport, bei der Hämofiltration (HF) liegt ein konvektiver Stofftransport über die Membran vor. Eine Kombination aus beiden Verfahren ist die Hämodiafiltration (HDF). Bei der Peritonealdialyse (PD) wird kein extrakorporaler Kreislauf benötigt und das Peritoneum als Kontaktmembran ausgenützt.

Wegen der großen Austauschmengen besteht bei den genannten Verfahren, sowie auch bei der kontinuierlichen arterio-venösen HF, der kontinuierlichen veno-venösen HF und der Plasmafiltration (PF) die Notwendigkeit der exakten Bilanzierung von entzogener Flüssigkeit einerseits zur zugeführten Flüssigkeit andererseits und der zu ultrafiltrierenden Menge über die gesamte Behandlungszeit. Zum Stand der Technik gehören gravimetrische und volumetrische Bilanziersysteme.

DE 41 16 178 C1 beschreibt eine Vorrichtung zur Reinigung von Blut mit volumetrischer Flüssigkeitsbilanzierung für HF-, HDF- und PF-Systeme. Die bekannte Blutbehandlungsvorrichtung weist eine Bilanzierkammer auf, die durch eine flexible Wand in eine erste und eine zweite Bilanzierkammerhälfte geteilt ist, wobei die erste Kammerhälfte mit einer Filtratleitung und die zweite Kammerhälfte mit einer Substituatleitung verbunden ist. Von der ersten Kammerhälfte geht eine Filtratauslaßleitung zu einem Auslaß ab, während von der zweiten Kammerhälfte eine Substituatauslaßleitung abgeht, die mit dem Blutkreislauf verbunden ist. Filtrat und Substituat werden der Bilanzierkammer wechselweise jeweils aus einer in die Filtrat- bzw. Substituatleitung geschalteten Ausgleichskammer mittels einer Druckeinrichtung zugeführt. Die Ausgleichskammern werden bei der bekannten Filtrationseinrichtung mit der Filtratpumpe bzw. der Substituatpumpe gefüllt und nach Öffnen von Absperrklemmen mittels der Druckeinrichtung in eine der Bilanzierkammerhälften entleert, wobei sich gleichzeitig die andere Bilanzierkammerhälfte unter Druckeinwirkung in die jeweilige Auslaßleitung entleert. Die Bilanzierkammer und die Ausgleichskammern bilden ein mehrteiliges Set, das nach einmaliger Verwendung verworfen wird. Die Ausgleichskammern des Sets sind als flexible in die Filtrat- bzw. Substituatleitungen geschaltete Kunststoffbeutel ausgebildet, während das Gehäuse der Bilanzierkammer ein starrer Kunststoffkörper ist. Da das Disposable der bekannten Filtrationseinrichtung aus mehreren Einzelkomponenten besteht, ist die Handhabung erschwert. Ferner ist die Herstellung des mehrteiligen Disposables relativ kostspielig.

Die DE 34 28 828 C2 beschreibt eine Vorrichtung zum Fördern von Blut in einem extrakorporalen Kreislauf mit einer Pumpkammer, deren Pumpraum eine Dauermembran aufweist, die einen Hohlraum umschließt. In dem Hohlraum befindet sich eine Wechselmembran, die aus elastischem Material besteht und die Form eines Schlauchs hat, der an einem seiner Enden mit einem Anschlußstück für zwei Leitungen verbunden ist. Der Pumpraum der Pumpkammer steht über eine Leitung mit einer mechanischen Kolbenpumpe in Verbindung, um den Pumpraum mit einer Arbeitsflüssigkeit füllen zu können, so daß die schlauchförmige Wechselmembran komprimiert wird.

Der Erfindung liegt die Aufgabe zugrunde, ein Bilanzierdisposable zum Bilanzieren von Flüssigkeiten für eine medizinische Behandlungsvorrichtung zu schaffen, das sich einfach handhaben und kostengünstig herstellen läßt. Ferner liegt der Erfindung die Aufgabe zugrunde, eine medizinische Behandlungsvorrichtung mit einem Systemeinschub zur Aufnahme eines derartigen Bilanzierdisposables zu schaffen.

Die Lösung der Aufgabe erfolgt erfindungsgemäß mit den Merkmalen des Patentanspruchs 1 bzw. 8.

Das erfindungsgemäße Bilanzierdisposable ist als Folienbeutel ausgebildet und weist sowohl eine Bilanzierkammer als auch zwei mit den jeweiligen Kammerhälften der Bilanzierkammer in Fluidverbindung stehende Ausgleichskammern auf. Das Bilanzierdisposable besteht im wesentlichen aus zwei Folien, die unter Ausbildung der durch eine flexible Wand in die beiden Kammerhälften unterteilten Bilanzierkammer und der beiden Ausgleichskammern sowie der die einzelnen Kammern verbindenden Kanäle miteinander druckdicht verbunden sind.

Bei dem erfindungsgemäßen Bilanzierdisposable kann das Funktionsprinzip der volumetrischen Bilanzierung mit dem wechselseitigen Füllen und Auspressen der beiden Bilanzierkammerhälften beibehalten werden. Die feste Volumenvorgabe der Bilanzierkammerhälften wird durch einen entsprechenden Systemeinschub der Bilanziervorrichtung erreicht, in den das erfindungsgemäße Disposable passend eingelegt werden kann. Der Systemeinschub weist zwei starre Aufnahmekörper mit muldenförmigen Vertiefungen zum Über- bzw. Untergreifen der Bilanzierkammer des Disposables auf. Beim Befüllen einer der beiden Kammerhälften legen sich die äußeren Folien des Disposables an die Innenflächen der beiden Schalenhälften an, so daß ein bestimmtes Bilanziervolumen vorgegeben ist. In dem Systemeinschub sind auch die bekannten Kompressionseinrichtungen integriert, welche die in den ersten bzw. zweiten Einlaßkanal geschalteten Ausgleichskammern mit Druck beaufschlagen. Ferner erfolgt das wechselseitige Abklemmen der jeweiligen Kanäle mit Klemmeinrichtungen, die ebenfalls in dem Systemeinschub eingebaut sind.

Bei Verwendung des Bilanzierdisposables in der PD gilt das Prinzip entsprechend. Hierbei wird jedoch nicht kontinuierlich Substituat zu Filtrat bilanziert, sondern durch zusätzliche Klemmen und Verbindungen zuerst frisches Dialysat über beide Bilanzierkammerhälften gefördert und nach Verweilen im Bauchraum das verbrauchte Dialysat in gleicher Weise in den Abfluß gefördert, d.h. es erfolgt keine unmittelbare Verdrängung von Substituat durch Filtrat und umgekehrt. Die Mengenbilanz erfolgt dabei über Addition der Einzelmengen, die pro Kammerfüllung ab- bzw. zugeführt werden.

Das erfindungsgemäße Disposable läßt sich auf einfache Weise in einem einzigen Schweiß- oder Klebevorgang kostengünstig herstellen. Da das Disposable sämtliche Komponenten der Bilanziervorrichtung umfaßt, ist seine Handhabung erheblich vereinfacht. Das einteilige Disposable kann mit einem Handgriff in den entsprechenden Systemeinschub der medizinischen Behandlungsvorrichtung eingelegt bzw. nach Gebrauch gegen einen neuen Folienbeutel ausgetauscht werden. Als vorteilhaft erweist sich auch die sehr flache Bauweise, die es erlaubt, den flexiblen Folienbeutel als sterile Einheit in einer flachen Kunststofftasche anzubieten, die gegen Stoß unempfindlich ist und sich leicht stapeln und transportieren läßt.

Die Folien des Bilanzierdisposables können prinzipiell miteinander verschweißt oder verklebt werden. Vorzugsweise werden die Folien aber miteinander verschweißt, um eine druckdichte Verbindung zu erzielen.

Die flexible Wand, die die erste Kammerhälfte der Bilanzierkammer von der zweiten Kammerhälfte trennt, wird durch eine Zwischenfolie gebildet, die zwischen die erste und zweite Folie im Bereich des Bilanziervolumens eingelegt und mit den äußeren Folien unter Ausbildung der ersten und zweiten Kammerhälfte druckdicht verbunden, z.B. verschweißt oder verklebt ist. Durch geeignete Verschweißung in den Eingängen und den Auslässen und durch das Anlegen der Folien bei Überdruck werden zwei Bilanzierkammerhälften geschaffen, die durch die Zwischenfolie vollständig getrennt sind. Bei Füllung der einen Kammer wird automatisch die gleiche Flüssigkeitsmenge in die andere Kammer herausgepreßt.

Da sich beim Schweißen der Folien an den Rändern der Schweißnaht Materialrückstände bilden, ist ein relativ großer Anpreßdruck erforderlich, um die Einlaß- und Auslaßkanäle druckdicht abzuklemmen. Vorteilhafterweise sind daher zumindest im Bereich der Klemmstellen der Kanäle zwischen die Ober- und Unterfolie des Disposables flexible Kunststoffschläuche eingelegt und mit den äußeren Folien verschweißt oder verklebt. Die an ihrer Innenseite nahtlosen Kanäle lassen sich auf einfache Weise mit verhältnismäßig geringem Druck abklemmen. Die flexiblen Kunststoffschläuche verringern ferner die Gefahr, daß die geschweißten Folienkanäle schon bei geringem Unterdruck kollabieren und den Rückfluß unterbrechen. Die Abklemmung der Kanäle erfolgt vorteilhafterweise durch Stößel, die in dem einen Aufnahmekörper des Systemeinschubs vorgesehen sind und mit Klemmkanten zusammenwirken, die auf der gegenüberliegenden Seite in dem anderen Aufnahmekörper angeordnet sind.

Das Bilanzierdisposable ist vorzugsweise mit Durchbrüchen zur Aufnahme von Fixierungsstiften versehen, die in dem Systemeinschub integriert sein können. Auf diese Weise ist eine exakte Positionierung des Disposables zwischen den Aufnahmekörpern des Systemeinschubs sichergestellt. Innerhalb des Systemeinschubs wird das Disposable vorzugsweise an seinen Rändern gehalten. In den Aufnahmekörpern des Systemeinschubs sind daher vorteilhafterweise Klemmränder vorgesehen, die das Disposable von beiden Seiten ergreifen.

In einer vorteilhaften Ausführungsform ist ein schlangenförmiger Kanal in dem Disposable ausgebildet, der in die zweite Ausgleichskammer mündet. Der schlangenförmige Kanal ermöglicht es, die in die zweite Ausgleichskammer geleitete Flüssigkeit durch die Folien hindurch mittels Heizplatten zu temperieren, die von außen auf das Disposable aufgelegt werden. Die Heizplatten können Bestandteil der Aufnahmekörper des Systemeinschubs sein, wobei die Strömungsführung und Verwirbelung innerhalb der Folie durch eine entsprechende Struktur im Einschub gewährleistet werden kann.

In einer weiteren bevorzugten Ausführungsform weist das Bilanzierdisposable eine Pufferkammer auf, die in den zweiten Auslaßkanal geschaltet ist. Diese Pufferkammer erlaubt es, den bei Verwendung nur eines Bilanziervolumens auftretenden pulsativen Fluß in einen quasi kontinuierlichen Fluß zu wandeln. Die Pufferwirkung wird hierbei über eine in dem Systemeinschub vorgesehene und mit dem Bilanzierdisposable zusammenwirkende mechanische Kopplung erreicht, die beim Befüllen der ersten Ausgleichskammer die Pufferkammer entleert. Hierdurch wird die blutseitige Drucküberwachung vereinfacht und die Pulsation der Nadel verringert.

Die Bilanzierkammer, die wegen einer besseren Breitenausnutzung vorzugsweise eine ovale Form hat und die rechteckförmigen Ausgleichskammern sind vorzugsweise derart angeordnet, daß die Längsachse der Bilanzierkammer quer zu den Längsachsen der Ausgleichskammern verläuft. Dadurch ist eine platzsparende Anordnung der Kammern möglich.

Nachfolgend werden unter Bezugnahme auf die Zeichnungen zwei Ausführungsformen des erfindungsgemäßen Bilanzierdisposables und eine Ausführungsform des Systemeinschubs einer Blutbehandlungsvorrichtung zur Aufnahme des Disposables näher erläutert.

Es zeigen:
- Fig. 1: das erfindungsgemäße Bilanzierdisposable in der Draufsicht,
- Fig. 2: einen Schnitt durch die Bilanzierkammer des Bilanzierdisposables,
- Fig. 3a: einen Schnitt durch die zwischen den beiden Aufnahmekörpern des Systemeinschubs liegende Bilanzierkammer des Bilanzierdisposables, wobei die untere Kammerhälfte gefüllt und die obere Kammerhälfte entleert ist,
- Fig. 3b: einen Schnitt durch die zwischen den beiden Aufnahmekörpern des Systemeinschubs liegende Bilanzierkammer des Bilanzierdisposables, wobei die untere Kammerhälfte entleert und die obere Kammerhälfte gefüllt ist,
- Fig. 4: eine zweite Ausführungsform des Bildanzierdisposables mit einer integrierten Heizfolie, und
- Fig. 5: den aufgeklappten Systemeinschub der medizinischen Behandlungsvorrichtung mit den beiden Aufnahmekörpern zur Aufnahme des erfindungsgemäßen Bilanzierdisposables.

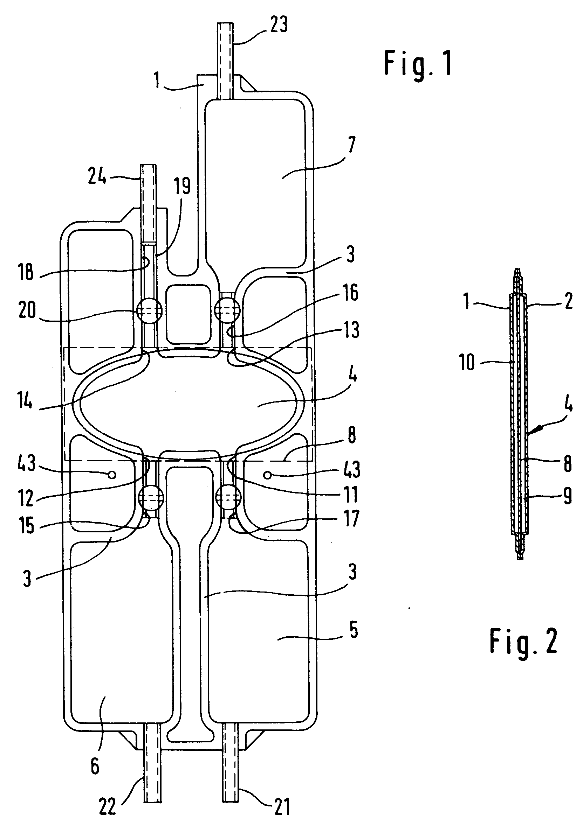

Fig. 1 zeigt das Bilanzierdisposable für eine medizinische Behandlungsvorrichtung. Das Bilanzierdisposable weist zwei flexible, im wesentlichen rechteckförmige Kunststoffolien 1, 2 auf, die entlang der mit dem Bezugszeichen 3 versehenen Nahtstellen derart miteinander verschweißt sind, daß zwischen der oberen und unteren Folie 1,2 insgesamt vier Kammern ausgebildet sind. Im Zentrum des Disposables befindet sich eine Bilanzierkammer 4 mit einer ovalen Form. Auf der einen Seite der Bilanzierkammer sind eine erste rechteckförmige Ausgleichskammer 5 und eine zweite rechteckförmige Ausgleichskammer 6 angeordnet, die das gleiche Aufnahmevolumen haben. Auf der anderen Seite der Bilanzierkammer 4 ist eine Pufferkammer 7 angeordnet.

Die Bilanzierkammer 4 weist eine flexible und elastische Wand 8 auf, die die Kammer in eine erste untere und eine zweite obere Kammerhälfte 9, 10 unterteilt, wobei jede Kammerhälfte 9, 10 mit einem Eingang 11, 12 und einem Ausgang 13, 14 versehen ist. Die flexible Wand 8 besteht aus einer in Fig. 1 durch gestrichelte Linien angedeutete rechteckförmige Zwischenfolie, die im Bereich des Bilanziervolumens zwischen die obere und untere Folie 1, 2 eingelegt ist und unter Ausbildung der beiden Kammerhälften 9, 10 mit den äußeren Folien 1, 2, verschweißt ist (Fig. 2).

Die untere Kammerhälfte 9 ist über einen ersten Einlaßkanal 17 mit der ersten Ausgleichskammer 5 in Fluidverbindung verbunden, während die obere Bilanzierkammerhälfte ist über einen zweiten Einlaßkanal 15 mit der zweiten Ausgleichskammer 6 und über einen schräg gegenüberliegenden zweiten Auslaßkanal 16 mit der Pufferkammer 7 in Fluidverbindung steht. An den Ausgang 14 der ersten Bilanzierkammerhälfte 9 ist ein erster Auslaßkanal 18 angeschlossen. Die Ein- und Auslaßkanäle 15 bis 18 zwischen den Kammern 4 bis 7 werden durch flexible Kunststoffschläuche 19 gebildet, die zwischen die obere und untere Folie 1, 2 eingelegt sind und mit den äußeren Folien verschweißt sind. Die Eingänge bzw. die Ausgänge der Schläuche 19 sind so mit der Zwischenfolie 8 der Bilanzierkammer 4 verschweißt, daß sich ein Eingang und ein Ausgang oberhalb und ein Eingang und ein Ausgang unterhalb der Zwischenfolie 8 befindet und eine direkte Verbindung zwischen der oberen und der unteren Bilanzierkammerhälfte 9, 10 nicht gegeben ist.

Die eingelegten Kunststoffschläuche 19 verhindern nicht nur ein Kollabieren der Schlauchkanäle bei Unterdruck, sondern schaffen auch in Fig. 1 durch Kreise dargestellte Klemmstellen 20, die sich druckdicht abklemmen lassen. Mit dem Eingang der ersten Ausgleichskammer 5 ist eine erste Zuleitung 21, mit dem Eingang der zweiten Ausgleichskammer 6 ist eine zweite Zuleitung 22, mit dem Ende des ersten Auslaßkanals 18 ist eine erste Auslaßleitung 24 und mit dem Ausgang der Pufferkammer 7 ist eine zweite Auslaßleitung 23 verschweißt. Die Ein- und Auslaßleitungen 21 bis 24 können mit in Fig. 1 nicht dargestellten Anschlußstücken versehen sein. Ferner weist das Bilanzierdisposable seitlich neben der Bilanzierkammer angeordnete Durchbrüche 43 zur Aufnahme von Fixierungsstiften auf. Dabei sind die Kammern jeweils als Beutelanordnungen zu verstehen.

Das erfindungsgemäße Bilanzierdisposable kann z.B. in einer Blutbehandlungsvorrichtung mit einer volumetrischen Flüssigkeitsbilanzierung Verwendung finden, die ein durch eine semipermeable Membran in eine Blutkammer und eine Filtratkammer unterteiltes Filter aufweist. Eine derartige extrakorporale Blutbehandlungsvorrichtung ist beispielsweise aus DE 41 16 178 C1 bekannt, auf die ausdrücklich Bezug genommen wird. Sofern das Bilanzierdisposable in einer derartigen Blutbehandlungsvorrichtung verwendet wird, bildet die erste Ausgleichskammer 5 die Filtratausgleichskammer und die zweite Ausgleichskammer 6 die Substituatausgleichskammer, während der erste und zweite Einlaßkanal 17, 15 den Filtrat- und Substituateinlaßkanal und der erste und zweite Auslaßkanal 18, 16 den Filtrat- und Substituatauslaßkanal bilden. Das erfindungsgemäße Bilanzierdisposable kann aber auch in vorteilhafter Weise in einer Vorrichtung zur Peritonealdialyse Verwendung finden, mit der zyklisch Peritonealdialysieiflüssigkeit in exakt bilanzierter Weise einem Patienten zugeführt bzw. entnommen werden kann. Hierzu werden die beiden Zuleitungen 21, 22 zu einer gemeinsamen Einlaßleitung zusammengeführt und die beiden Auslaßleitungen 23, 24 werden zu einer gemeinsamen Auslaßleitung zusammengeführt, wobei die Dialysierflüssigkeit über die gemeinsame Einlaßleitung wechselweise in die erste und zweite Bilanzierkammerhälfte 9, 10 geleitet wird und über die gemeinsame Auslaßleitung dem Peritonealraum des Patienten zugeführt wird. Bei einem derartigen Peritonealdialysegerät wird vorteilhafterweise eine Pufferkammer in den ersten und eine Pufferkammer in den zweiten Auslaßkanal geschaltet. Alternativ kann aber auch eine Pufferkammer nach der Zusammenführung in die gemeinsame Auslaßleitung geschaltet werden.

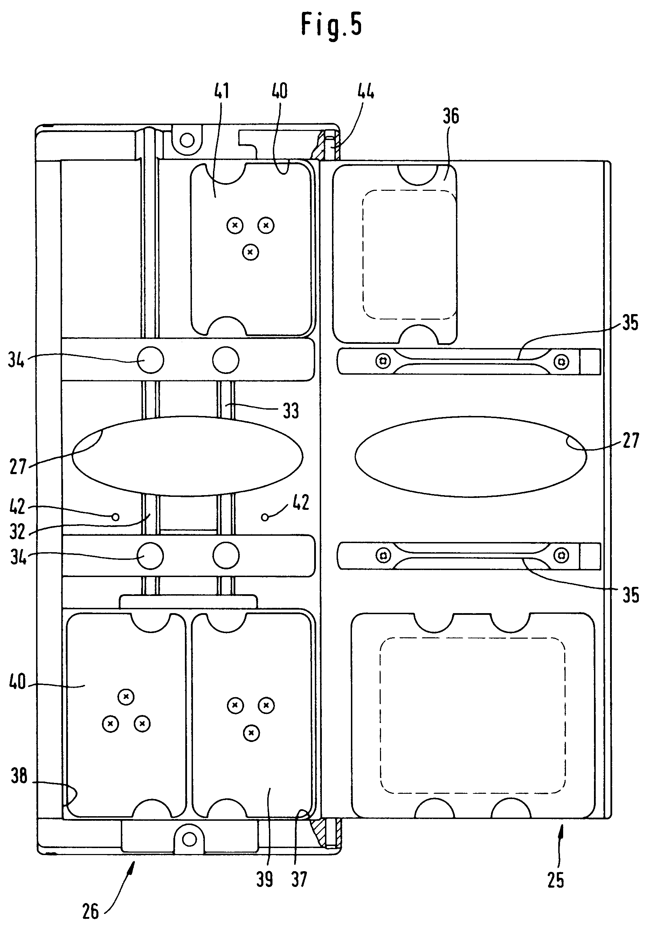

Fig. 2 zeigt einen Schnitt durch die Bilanzierkammer 4 des nicht mit Flüssigkeit gefüllten Bilanzierdisposables. Die Zwischenfolie 8 ist mit ihrem äußeren Rand mit den Rändern der Ober- und Unterfolie 1, 2 verschweißt und in der Bilanzkammer 4 zwischen den äußeren Folien frei beweglich. Da sich wegen der Elastizität der Kunststoffolien ein konstantes Volumen nicht genau einhalten läßt, wird das Bilanziervolumen durch eine feste äußere Form definiert. Das Bilanzierdisposable wird daher in einen Systemeinschub eingesetzt, der Bestandteil der Blutbehandlungvorrichtung ist. Der im einzelnen noch unter Bezugnahme auf Fig. 5 beschriebene Systemeinschub weist zwei Aufnahmekörper 25, 26 mit jeweils einer im Bereich des Bilanziervolumens vorgesehenen Vertiefung 27 auf.

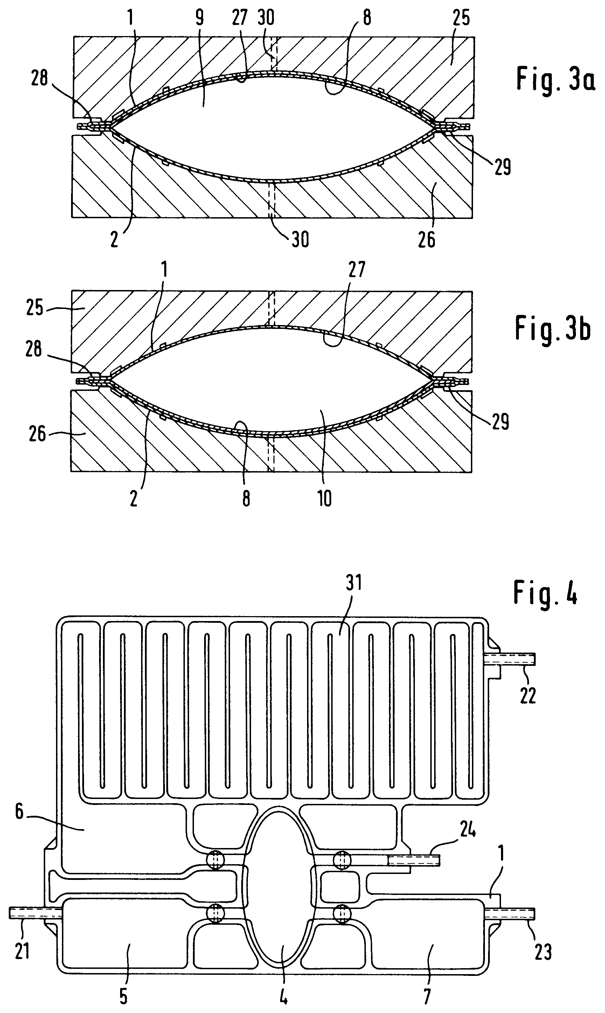

Die Fign. 3a und 3b zeigen einen Schnitt durch die zwischen den beiden Aufnahmekörpern 25, 26 des Systemeinschubs liegende Bilanzierkammer 4 des Disposables. Der obere und untere Aufnahmekörper 25, 26 des Systemeinschubs ist mit einem längslaufenden Klemmrand 28, 29 versehen, der die Randbereiche des Bilanzierdisposables fixiert. Wird das Volumen zwischen der Oberfolie 1 und der Zwischenfolie 8 mit Flüssigkeit gefüllt, so wird bei entsprechendem Öffnen und Schließen der Ein- und Ausgänge der Bilanzierkammer 4 durch Umschlagen der Zwischenfolie 8 das andere Volumen zwischen der Zwischenfolie 8 und der Unterfolie 2 infolge der Verdrängung entleert (Fig. 3b). Damit sich die Ober- und Unterfolie 1, 2 des Disposables dicht an die Schalen der Aufnahmekörper 25, 26 anlegen können, sind in den Aufnahmekörpern Entlüftungsöffnungen 30 vorgesehen.

Fig. 4 zeigt eine zweite Ausführungsform des Bilanzierdisposables. Das in Fig. 5 dargestellte Bilanzierdisposable unterscheidet sich von dem unter Bezugnahme auf die Fign. 1 bis 3 beschriebenen Disposables dadurch, daß ein schlangenförmiger Kanal 31 ausgebildet ist, der in die zweite Ausgleichskammer 6 mündet und eine Temperierung der zugeführten Flüssigkeit ermöglicht. Die Erwärmung des Fluids erfolgt durch die Heizfolien hindurch mittels in einem entsprechenden Systemeinschub vorgesehener Heizplatten, die mit der Heizfolie des Disposables in Kontakt gebracht werden. Zur Regelung und Temperaturüberwachung können in Fig. 4 nicht dargestellte Temperatursensoren vorgesehen sein, die auf einfache Weise die Temperatur an den Oberflächen der beiden äußeren Folien 1, 2 messen können. Zweckmäßigerweise wird zumindest ein Regel- und ein Schutzsensor stromab der Heizfolie angeordnet. Zusätzlich kann ein weiterer Temperatursensor am Heizfolieneingang vorteilhaft sein.

Fig. 5 zeigt den aufgeklappten Systemeinschub der medizinischen Behandlungsvorrichtung in der Draufsicht, in den das Bilanzierdisposable passend eingelegt wird. Der obere und untere in seinen äußeren Abmessungen dem Disposable entsprechende Aufnahmekörper 25, 26 des Systemeinschubs sind mit einem Scharnier 44 an ihren Längsseiten miteinander verbunden. Die Vertiefungen 27 zur Aufnahme der Bilanzierkammerhälften 9, 10 sind in Zentrum der beiden Aufnahmekörper 25, 26 angeordnet. Die Fixierung des Disposables erfolgt mit den Führungsstiften 42, die in die entsprechenden Durchbrüche 43 des Disposables greifen. Zu beiden Seiten der Vertiefungen 27 sind in dem unteren Aufnahmekörper 26 jeweils zwei parallele Führungskanäle 32, 33 zur Aufnahme der Ein- und Auslaßkanäle 15-18 vorgesehen. Ferner sind innerhalb der Führungskanäle 32, 33 vier Stößel 34 angeordnet, die mit den Klemmkanten 35 zusammenwirken, die sich auf der Höhe der Stößel 34 in dem oberen Aufnahmekörper 25 befinden. Mit den elektromagnetisch betätigbaren Stößeln 34 können die Klemmstellen 20 der Kanäle des Disposables druckdicht abgeklemmt werden. Seitlich neben den Stößeln 34 bzw. den Klemmkanten 35 sind in dem unteren und oberen Aufnahmekörper 25, 26 Einsatzstücke 36, 37, 38 zur Aufnahme der ersten- bzw. der zweiten Ausgleichskammer 5, 6 sowie der Pufferkammer 7 vorgesehen. Am Boden der Einsatzstücke 37, 38 für die Ausgleichskammern sind die federnd vorgespannten Druckplatten 39, 40 der in dem Systemeinschub befindlichen Kompressionseinrichtungen angeordnet, mit denen die Ausgleichskammern 5, 6 des Disposables mit Druck beaufschlagt werden. Die Funktion der Klemmeinrichtungen und der Kompressionseinrichtungen ist in DE 41 16 178 C1 beschrieben, auf die ausdrücklich Bezug genommen wird. Am Boden des Einsatzstücks 40 für die Pufferkammer 7 ist eine weitere Druckplatte 41 vorgesehen. Diese kann mit der Druckplatte 39 der ersten Ausgleichskammer derart mechanisch gekoppelt sein, daß sie eine gegenläufige Bewegung ausübt.

## Patentansprüche

1. Bilanzierdisposable zum Bilanzieren von Flüssigkeiten für eine medizinische Behandlungsvorrichtung mit zwei übereinandergelegten Kunststoffolien (1, 2), die miteinander druckfest verbunden sind unter Bildung
einer durch eine flexible Wand (8) in eine erste und eine zweite Kammerhälfte (9, 10) unterteilten Bilanzierkammer (4), wobei die flexible Wand (8) der Bilanzierkammer (4) durch eine Zwischenfolie gebildet wird, die zwischen der ersten und zweiten Folie (1, 2) im Bereich des Bilanziervolumens eingelegt und mit den äußeren Folien (1, 2) unter Bildung der beiden Kammerhälften (9, 10) derart verbunden ist, daß die erste Kammerhälfte (9) von der zweiten Kammerhälfte (10) druckdicht getrennt ist,
einer über einen druckdicht abklemmbaren ersten Einlaßkanal (17) mit der ersten Kammerhälfte (9) der Bilanzierkammer (4) in Fluidverbindung stehenden ersten Ausgleichskammer (5),
einer über einen druckdicht abklemmbaren zweiten Einlaßkanal (15) mit der zweiten Kammerhälfte (10) der Bilanzierkammer (4) in Fluidverbindung stehenden zweiten Ausgleichskammer (6) und
eines mit der ersten Kammerhälfte (9) der Bilanzierkammer (4) in Fluidverbindung stehenden druckdicht abklemmbaren ersten Auslaßkanals (18) und eines mit der zweiten Kammerhälfte (10) der Bilanzierkammer (4) in Fluidverbindung stehenden druckdicht abklemmbaren zweiten Auslaßkanals (16).

2. Bilanzierdisposable nach Anspruch 1, **dadurch gekennzeichnet,** daß die Einlaß- und Auslaßkanäle (15-18) zumindest im Bereich ihrer Klemmstellen (20) durch zwischen der ersten und zweiten Folie (1, 2) angeordnete flexible Kunststoffschläuche (19) gebildet werden, die mit den äußeren Folien (1, 2) druckdicht verbunden sind.

3. Bilanzierdisposable nach einem der Ansprüche 1 oder 2, **dadurch gekennzeichnet,** daß das Bilanzierdisposable mit Durchbrüchen (43) zur Aufnahme von Fixierungsstiften (42) versehen ist.

4. Bilanzierdisposable nach einem der Ansprüche 1 bis 3, **dadurch gekennzeichnet,** daß die erste und zweite Folie (1, 2) miteinander druckdicht verbunden sind, ferner unter Ausbildung eines schlangenförmigen Kanals (31), der in die zweite Ausgleichskammer (6) mündet.

5. Bilanzierdisposable nach einem der Ansprüche 1 bis 4, **dadurch gekennzeichnet,** daß die erste und zweite Folie (1, 2) miteinander druckdicht verbunden sind, ferner unter Ausbildung einer in den zweiten Auslaßkanal (16) geschalteten Pufferkammer (7).

6. Bilanzierdisposable nach einem der Ansprüche 1 bis 5, **dadurch gekennzeichnet,** daß die Bilanzierkammer (4) eine ovale Form und die Ausgleichskammern (5, 6) eine rechteckförmige Form aufweisen, wobei die Ausgleichskammern (5, 6) und die Bilanzierkammer (4) derart angeordnet sind, daß die Längsachsen der Ausgleichskammern quer zur Längsachse der Bilanzierkammer verlaufen.

7. Medizinische Behandlungsvorrichtung zur Entfernung von toxischen Stoffen aus Blut mit einem Systemeinschub zur Aufnahme des Bilanzierdisposables nach einem der Ansprüche 1 bis 7, wobei der Systemeinschub zwei starre jeweils im Bereich der Bilanzierkammer (4) des Bilanzierdisposables mit einer muldenförmigen Vertiefung (27) versehene Aufnahmekörper (25, 26) und Kompressionseinrichtungen (39, 40) zum Beaufschlagen der Ausgleichskammern (5, 6) des Disposables mit Druck und Klemmeinrichtungen (34, 35) zum Abdrücken der Einlaß- und Auslaßkanäle (15-18) aufweist.

8. Blutbehandlungsvorrichtung nach Anspruch 7, **dadurch gekennzeichnet,** daß die Aufnahmekörper (25, 26) jeweils einen Klemmrand (28, 29) zum Fixieren der Randbereiche des Foliendisposables aufweisen.

9. Blutbehandlungsvorrichtung nach Anspruch 7 oder 8, **dadurch gekennzeichnet,** daß die Klemmeinrichtungen in dem einen Aufnahmekörper (26) vorgesehene Stößel (34) und in dem anderen Aufnahmekörper (25) vorgesehene den Stößeln (34) gegenüberliegende Klemmkanten (35) aufweisen.
